(19) Europäisches Patentamt
European Patent Office
Office européen des brevets

(11) Veröffentlichungsnummer: **0 336 186 A2**

# (12) EUROPÄISCHE PATENTANMELDUNG

(21) Anmeldenummer: 89104904.1

(22) Anmeldetag: 18.03.89

(51) Int. Cl.4: A01N 43/653, A01N 47/02, B27K 3/34

(30) Priorität: 02.04.88 DE 3811302

(43) Veröffentlichungstag der Anmeldung: 11.10.89 Patentblatt 89/41

(84) Benannte Vertragsstaaten: BE CH DE FR GB IT LI NL SE

(71) Anmelder: BAYER AG
D-5090 Leverkusen 1 Bayerwerk(DE)

(72) Erfinder: Bielefeldt, Dietmar, Dr.
Beuthener Strasse 13
D-4030 Ratingen 6(DE)
Erfinder: Böckmann, Klaus, Dr.
Claudius-Weg 7
D-5600 Wuppertal(DE)
Erfinder: Frie, Monika, Dr.
Im alten Driesch
D-5068 Odenthal(DE)
Erfinder: Himmler, Thomas, Dr.
Bonhoefferstrasse 20
D-5000 Köln 80(DE)
Erfinder: Paulus, Wilfried, Dr.
Deswatines-Strasse 90
D-4150 Krefeld 1(DE)
Erfinder: Regel, Erik
Untere Bergerheide 26
D-5600 Wuppertal(DE)
Erfinder: Schmitt, Hans-Georg, Dr.
Am Oberend 13
D-4150 Krefeld 1(DE)
Erfinder: Stroech, Klaus, Dr.
Rolsberger Strasse 22
D-5650 Solingen 19(DE)

## (54) Derivate des Triazolylmethylcyclopropyl-carbinols als Materialschutzmittel.

(57) Die Erfindung betrifft die Verwendung von Derivaten des Triazolylmethylcyclopropyl-carbinols der Formel

EP 0 336 186 A2

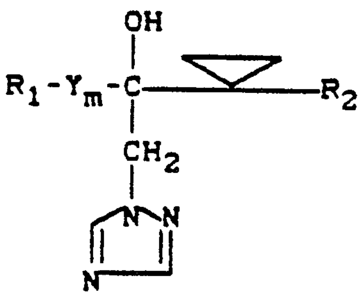

(I),

in der

$R_1$ eine gegebenenfalls substituierte Alkyl-, Cycloalkyl- oder Arylgruppe und

Y eine $-CH_2-$, $-OCH_2-$, $-CH_2-CH_2-$ oder $-CH=CH$-Gruppe bedeuten,

m für 0 oder 1 steht und

$R_2$ Halogen oder die Gruppe $ZR_4$ bedeutet, in der Z für Sauerstoff, Schwefel, eine Sulfinyl-oder Sulfonylgruppe steht und $R_4$ ein gegebenenfalls durch Halogen substituierter Alkyl-, Cycloalkyl-, Aralkyl- oder Aryl-Rest ist,

als Mikrobizide zum Schutz technischer Materialien.

## Derivate des Triazolylmethyl-cyclopropyl-carbinols als Materialschutzmittel

Die Erfindung betrifft die Verwendung von Derivaten des Triazolylmethyl-cyclopropyl-carbinols als Mikrobizide für den Materialschutz.

Es ist bekannt, daß Derivate des Triazolylmethyl-cyclopropyl-carbinols gegen bestimmte phyto- und humanpathogene Pilze wirksam sind (siehe EP-A-0 040 345, 0 180 136, 0 180 850 und DE-OS 36 17 190). Weiterhin ist bekannt, daß Triazolylverbindungen wie Triadimefon, Triadimenol, Diclobutrazol, die wegen ihrer guten selektiven Wirkung zum Schutz von Pflanzen und Saatgut verwendet werden, im Materialschutz nicht einsetzbar sind, weil ihr Wirkungsspektrum für dieses Anwendungsgebiet zu eng ist (siehe EP-A-0 180 313). Während im Pflanzenschutz und in der Human- und Veterinärmedizin möglichst selektiv wirkende Fungizide gefragt sind, erfordert der Materialschutz, da technische Materialien stets dem Angriff einer großen Zahl verschiedener Pilzarten ausgesetzt sind, Fungizide, die ein möglichst breites Wir kungsspektrum aufweisen. Da ohne breites Wirkungsspektrum kein zuverlässiger Schutz technischer Materialien gewährleistet ist, sind im Materialschutz nur Fungizide mit breitem Wirkungsspektrum verwendbar oder - ersatzweise - Fungizidkombinationen, die so zusammengestellt sind, daß sich die schmalen Wirkungsbereiche ihrer einzelnen Komponenten zu einem breiten Wirkungsspektrum ergänzen (siehe z.B. EP-A-0 180 313).

Es wurde nun gefunden, daß bestimmte Derivate des Triazolylmethyl-cyclopropyl-carbinols nicht nur eine ausgezeichnete fungizide Wirkung sondern gleichzeitig ein überraschend breites Wirkungsspektrum aufweisen und deshalb mit Vorteil als Mikrobizide zum Schutz technischer Materialien verwendet werden können.

Die Erfindung betrifft daher die Verwendung von Derivaten des Triazolylmethyl-cyclopropyl-carbinols der Formel

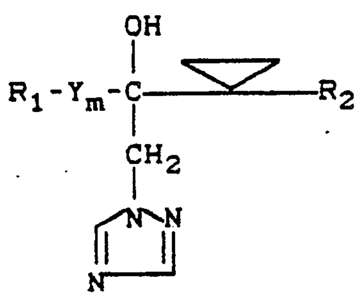

(I)

in der

$R_1$ eine gegebenenfalls substituierte Alkyl-, Cycloalkyl- oder Arylgruppe und

Y eine $-CH_2-$, $-OCH_2-$, $-CH_2-CH_2-$ oder $-CH=CH-$Gruppe bedeuten,

m für 0 oder 1 steht und

$R_2$ Halogen oder die Gruppe $ZR_4$ bedeutet, in der Z für Sauerstoff, Schwefel, eine Sulfinyl- oder Sulfonylgruppe steht und $R_4$ ein gegebenenfalls durch Halogen substituierter Alkyl-, Cycloalkyl-, Aralkyl- oder Aryl-Rest ist,

als Mikrobizide zum Schutz technischer Materialien.

Besonders bevorzugt sind die Verbindungen der Formel (I), in der

$R_1$ ein gegebenenfalls durch Halogen, Alkyl oder Halogenalkyl substituierter Phenyl-, Biphenyl-oder Naphthylrest ist,

$R_2$ für Halogen oder eine gegebenenfalls durch Halogen substituierte Alkylmercapto-Gruppe steht und

Y und m die unter Formel (I) angegebene Bedeutung haben.

Für $R_1$ seien als gegebenenfalls substituierte Alkylreste vorzugsweise durch Halogen, z.B. Fluor oder Chlor sub stituierte $C_1$-$C_4$-Alkylreste, als gegebenenfalls substituierte Cycloalkylreste durch Halogen, wie Chlor oder Brom, und/oder $C_1$-$C_4$-Alkylgruppen substituierte Cyclohexylreste und als gegebenenfalls substituierte Arylreste durch Halogen, vorzugsweise Fluor oder Chlor, durch $C_1$-$C_4$-Alkyl-, -Alkoxy- und -Alkylmercaptogruppen, durch Halogen wie Fluor und/oder Chlor, substituierte $C_1$-$C_4$-Alkyl-, -Alkoxy- und -Alkylmercaptogruppen und/oder durch Phenoxygruppen substituierte Phenyl-, Biphenyl-und Naphthylreste genannt.

Als Vertreter solcher gegebenenfalls durch Halogen oder Alkyl oder durch Halogen substituiertes Alkyl, Alkoxy, Alkylmercapto oder Phenoxy substituierter Phenylgruppen seien beispielsweise die folgenden

Phenylreste genannt: der Phenylrest, der 4-Chlor-, 4-Brom-, 4-Fluor-, 4-Trifluormethoxy- und 4-Trifluormethylmercapto-phenylrest, der 2,4- und 3,4-Difluorphenylrest, der 3,4-Dichlorphenylrest, der 4-Phenoxyphenylrest und der 4-Methyl-phenylrest.

Für $R_2$ seien als Halogen vor allem Chlor und Brom genannt; als Vertreter der Gruppe $ZR_4$ kommen vor allem gegebenenfalls durch Halogen substituierte $C_1$-$C_4$-Alkyl-mercaptogruppen wie die Methylmercapto-, die Dichlorfluormethylmercapto- und die Trifluormethylmercaptogruppe in Betracht.

Als Vertreter der erfindungsgemäß zu verwendenden Verbindungen seien solche Verbindungen der Formel (I) genannt, in der $R_1$, Y, m und $R_2$ folgende Bedeutungen haben:

| $R_1$ | Y | m | $R_2$ |
|---|---|---|---|
| 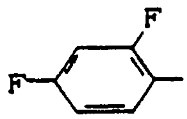 | - | 0 | 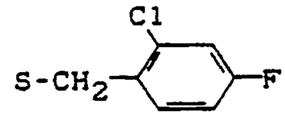 |
| 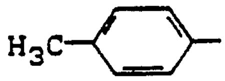 | - | 0 | $SCF_3$ |
| 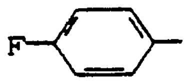 | - | 0 | $SCF_3$ |
| 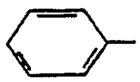 | - | 0 | $SCF_3$ |
| 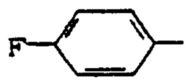 | - | 0 | Br |
| 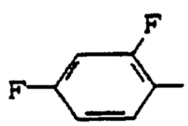 | - | 0 | Br |
| 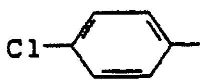 | $-CH_2-CH_2-$ | 1 | Cl |
| 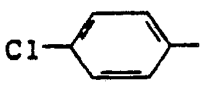 | $-CH_2-CH_2-$ | 1 | 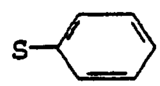 |
| 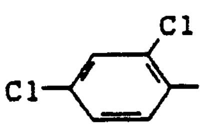 | -CH=CH- | 1 | $SC_3H_7$ |

| $R_1$ | Y | m | $R_2$ |
|---|---|---|---|
| 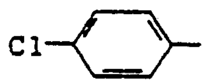 | $-CH_2-CH_2-$ | 1 | 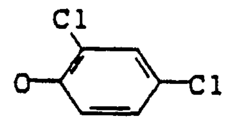 |
| 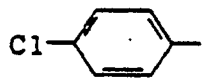 | $-CH_2-CH_2-$ | 1 | $SCH_3$ |
| 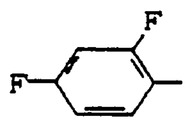 | $-CH_2-CH_2-$ | 1 | Cl |
| 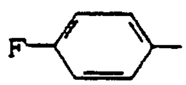 | $-CH_2-CH_2$ | 1 | Cl |
| 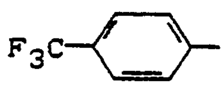 | $-CH_2-CH_2-$ | 1 | Cl |
| 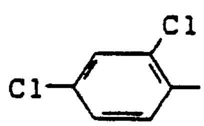 | -CH=CH- | 1 | Cl |
| 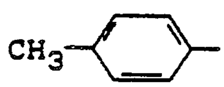 | $-CH_2-CH_2-$ | 1 | Cl |
| 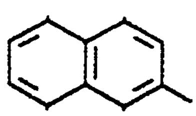 | -CH=CH- | 1 | Cl |
| 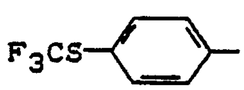 | -CH=CH- | 1 | Cl |

| $R_1$ | Y | m | $R_2$ |
|---|---|---|---|
| 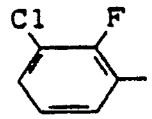 | -CH=CH- | 1 | Cl |
| 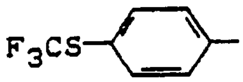 | $-CH_2-CH_2-$ | 1 | Cl |
| 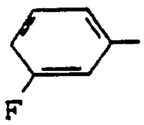 | $-CH_2-CH_2-$ | 1 | Cl |
| 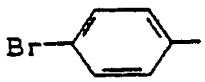 | $-CH_2-CH_2-$ | 1 | Cl |
| 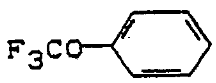 | -CH=CH- | 1 | Cl |
| 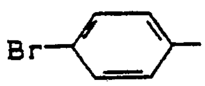 | -CH=CH- | 1 | Cl |
| 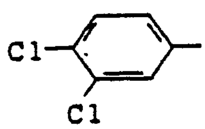 | -CH=CH- | 1 | Cl |
| 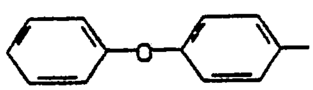 | - | 0 | Cl |
| 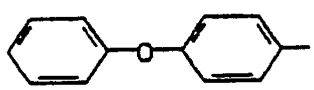 | $-CH_2-$ | 1 | Cl |

Die erfindungsgemäß zu verwendenden Derivate des Triazolylmethyl-cyclopropyl-carbinols können als solche oder in Form ihrer Salze eingesetzt werden; zur Salzbildung können Säuren wie Halogenwasserstoffsäuren, z.B. Chlorwasserstoffsäure, ferner Phosphorsäure, Schwefelsäure und mono- und bifunktionelle Carbonsäuren wie Essigsäure, Bernsteinsäure und Milchsäure, ferner Verbindungen mit aciden Wasserstoffatomen wie Saccharin, verwendet werden.

Die Herstellung der erfindungsgemäß zu verwendenden Verbindungen der Formel (I) in der m für 0 steht, ist in der EP-A-0 180 136 beschrieben.

Die Herstellung der erfindungsgemäß zu verwendenden Verbindungen der Formel (I), in der m den Wert 1 und Y und $R_2$ eine der angegebenen Bedeutungen haben, erfolgt in folgender Weise:

Für Verbindungen der Formel (I), in der Y eine $CH_2$-$CH_2$-Gruppe, m = 1 und $R_2$ Halogen ist, nach folgendem Reaktionsschema:

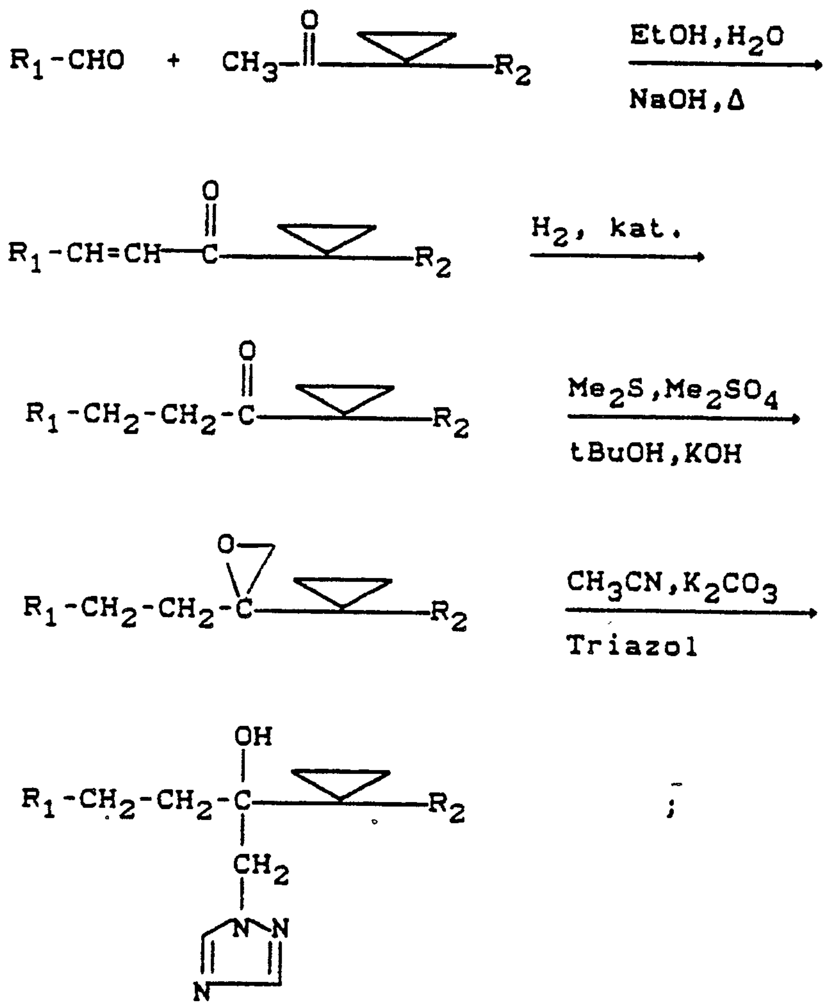


für Verbindungen der Formel (I), in der Y eine -CH=CH-Gruppe, m = 1 und $R_2$ Halogen ist, nach folgendem Reaktionsschema:

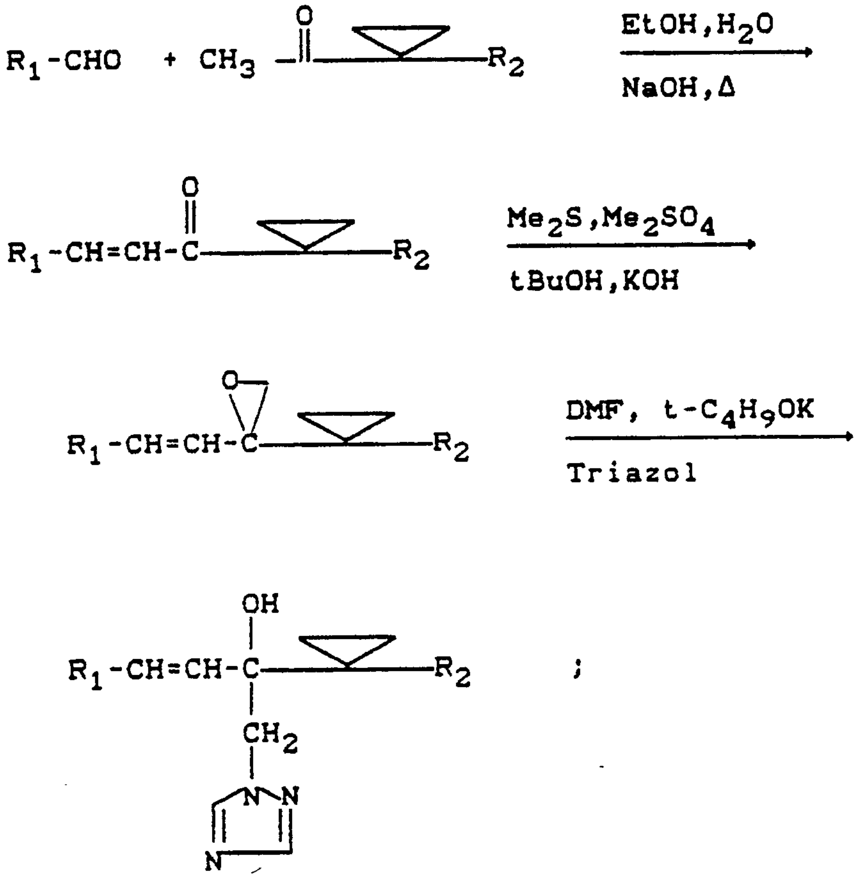


;

für Verbindungen der Formel (I), in der Y eine $-CH_2$-Gruppe, m = 1 und $R_2$ Halogen ist, nach folgendem Reaktionsschema:

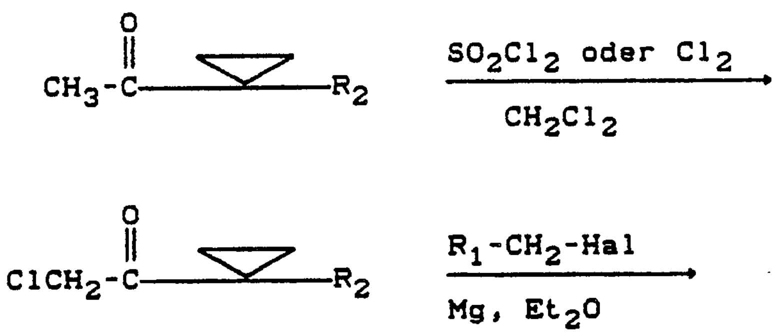

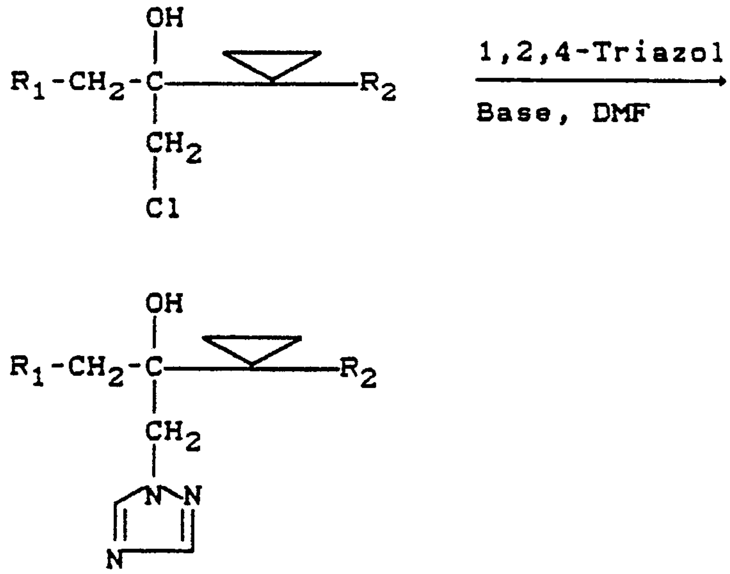


(Als Base werden z.B. Kalium-tert.-butylat oder Kaliumcarbonat verwendet).

Für Verbindungen der Formel (I), in der $R_1$ ein gegebenenfalls substituierter Phenyl-, Biphenyl- oder Naphthylrest, Y eine $-OCH_2$-Gruppe, m = 1 und $R_2$ Halogen ist, nach folgendem Reaktionsschema:

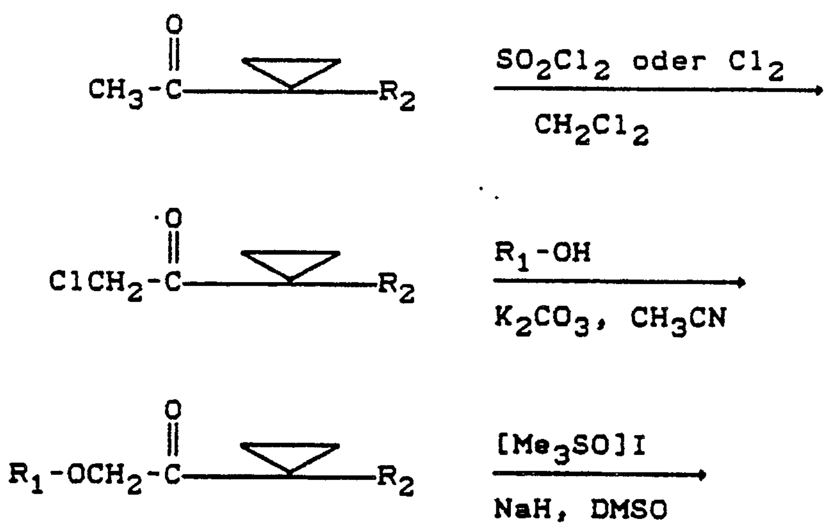

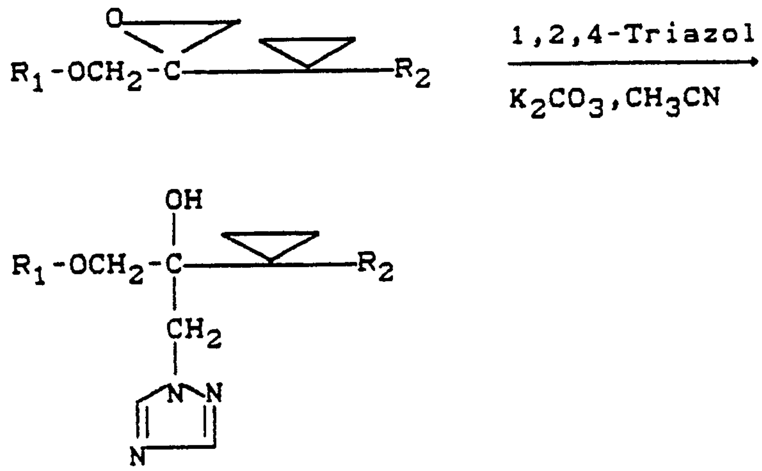


für Verbindungen der Formel (I), in der m = 0, $R_2$ = $SR_4$ und $R_4$ ein Perfluoralkylrest ist, nach folgendem Reaktionsschema:

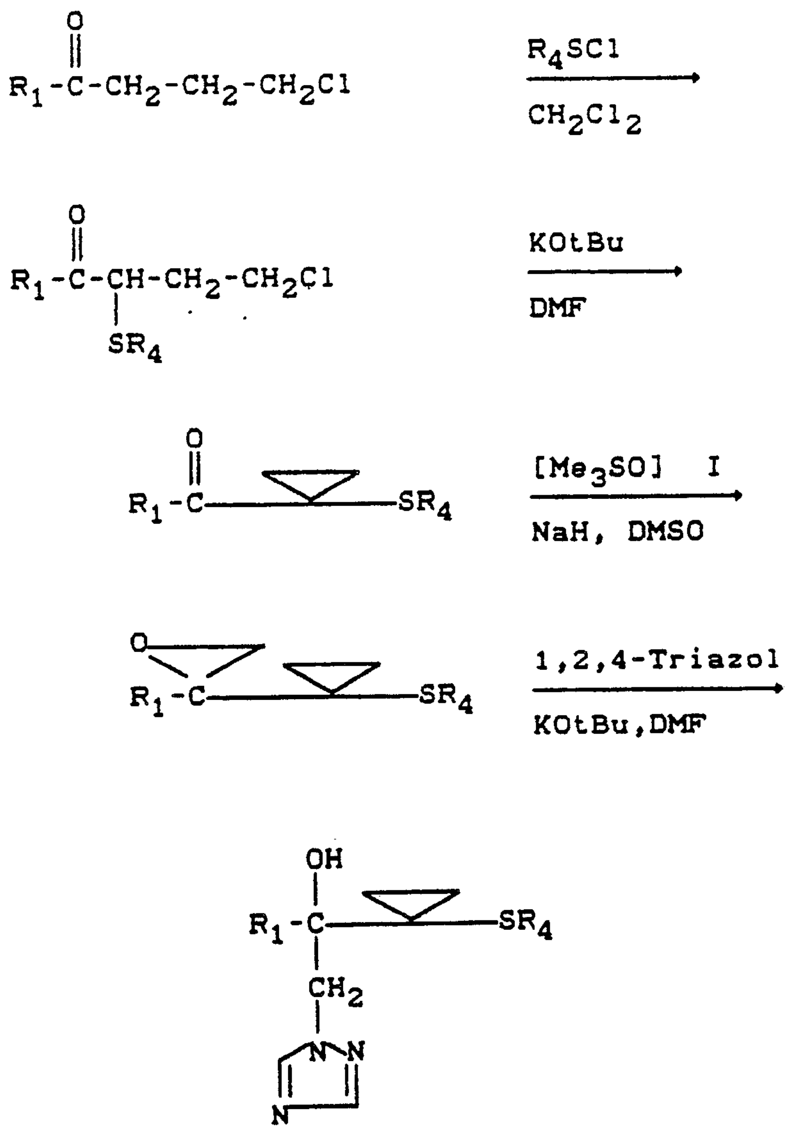


Bei den erfindungsgemäß zu schützenden technischen Materialien handelt es sich um nicht lebende

Materalien, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel, Kunststoffe, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, deren Funktion durch Vermehrung von Mikroorganismen beeinträchtigt werden kann. Im Rahmen der vorliegenden Erfindung seien als technische Materialien vorzugsweise Kunststoffe, Leder, Holz, Anstrichmittel, Kühlschmiermittel und Wärmeübertragungsflüssigkeiten genannt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, Holz dauerhaft verfärbende und holzzerstörende Pilze, sowie gegen Schleimorganismen und Algen.

Es seien beispielsweise Mikroorganismen der folgenden Gattungen genannt:

Alternaria, wie Alternaria tenuis,
Aspergillus, wie Aspergillus niger,
Chaetomium, wie Chaetomium globosum,
Coniophora, wie Coniophora puteana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Je nach Anwendungsgebiet können die erfindungsgemäß zu verwendenden Wirkstoffe in die üblichen Formulierungen überführt werden, wie Lösungen, Emulsionen, Suspensionen, Pulver, Pasten und Granulate.

Diese können in an sich bekannter Weise hergestellt werden, z.B. durch Vermischen der Wirkstoffe mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei gegebenenfalls im Falle der Benutzung von Wasser als Streckmittel organische Lösungsmittel wie Alkohole als Hilfsmittel verwendet werden können.

Flüssige Lösungsmittel für die Wirkstoffe können beispielsweise Wasser, Alkohole, wie niedere aliphatische Alkohole, vorzugsweise Ethanol oder Isopropanol, oder Benzylalkohol, Ketone, wie Aceton oder Methylethylketon, flüssige Kohlenwasserstoffe, wie Benzinfraktionen, halogenierte Kohlenwasserstoffe, wie 1,2-Dichlorethan, sein.

Die zum Schutz technischer Materialien verwendeten mikrobiziden Mittel enthalten die Wirkstoffe im allgemeinen in einer Menge von 1 bis 95 Gew.-%, bevorzugt von 10 bis 75 Gew.-%.

Die Anwendungskonzentrationen der erfindungsgemäß zu verwendenden Wirkstoffe richten sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Die erfindungsgemäß zu verwendenden Wirkstoffe können auch in Mischung mit anderen bekannten Wirkstoffen angewendet werden. Beispielsweise seien die folgenden Wirkstoffe genannt: Benzylalkoholmono(poly)hemiformal und andere Formaldehyd abspaltende Verbindungen, Benzimidazolylmethylcarbamate, Tetramethylthiuramdisulfid, Zinksalze von Dialkyldithiocarbamaten, 2,4,5,6-Tetrachlorisophthalonitril, Thiazolylbenzimidazol, Mercaptobenzthiazol, 2-Thiocyanatomethylthiobenzthiazol, Methylenbisthiocyanat, Phenolderivate, wie 2-Phenylphenol, (2,2′-Dihydroxy-5,5′-dichlor)-diphenylmethan und 3-Methyl-4-chlor-phenol, Organo-Zinnverbindungen, N-Trihalogenmethylthio-verbindungen, wie Folpet, Fluorfolpet, Dichlofluanid.

In den nachfolgenden Beispielen wurden solche Verbindungen der Formel (I) verwendet, in der $R_1$, Y, m und $R_2$ die folgende Bedeutung haben:

| Verbindung | $R_1$ | Y | m | $R_2$ | Fp.: |
|---|---|---|---|---|---|
| 1 | 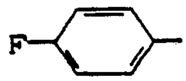 | - | 0 | Br | 123° C |
| 2 | 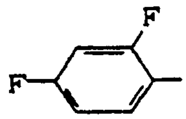 | - | 0 | Br | 115° C |
| 3 | 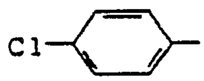 | - | 0 | Cl | 139° C |
| 4 | 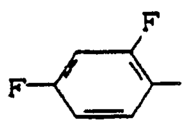 | - | 0 | $-SCF_3$ | 103° C |
| 5 | 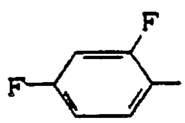 | - | 0 | $-SCH_3$ | 89° C |
| 6 | 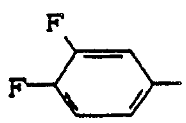 | - | 0 | $-SCH_3$ | 121° C |
| 7 | 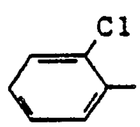 | $-CH_2-$ | 1 | Cl | 108° C |
| 8 | 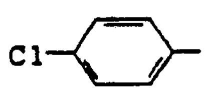 | $-CH_2-$ | 1 | Cl | 97° C |

| Verbindung | $R_1$ | Y | m | $R_2$ | Fp.: |
|---|---|---|---|---|---|
| 9 | 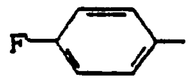 | $-CH_2-$ | 1 | Cl | 111° C |
| 10 | 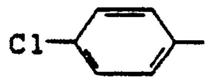 | $-CH_2-CH_2$ | 1 | Cl | 137° C |
| 11 | 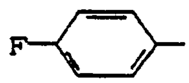 | $-CH_2-CH_2-$ | 1 | Cl | Öl |
| 12 | 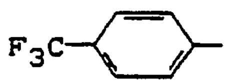 | $-CH_2-CH_2-$ | 1 | Cl | Öl |
| 13 | 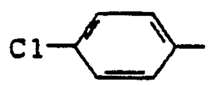 | $-OCH_2-$ | 1 | Cl | Öl |
| 14 | 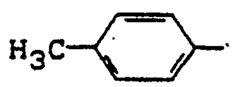 | $-CH_2-CH_2$ | 1 | Cl | 118° C |
| 15 | 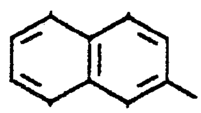 | -CH=CH- | 1 | Cl | 116° C |
| 16 | 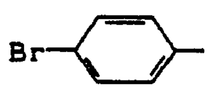 | -CH=CH- | 1 | Cl | 130° C |
| 17 | 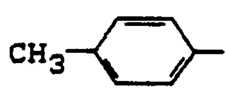 | -CH=CH- | 1 | Cl | 106° C |

| Verbindung | $R_1$ | Y | m | $R_2$ | Fp.: |
|---|---|---|---|---|---|
| 18 | 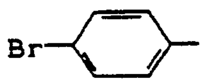 | $-CH_2-CH_2-$ | 1 | Cl | 132° C |
| 19 | 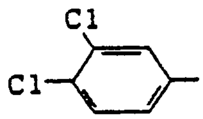 | -CH=CH- | 1 | Cl | 140° C |

Die Verbindungen 1-3, 5 und 6 wurden nach dem in der EP-A-0 180 136 angegebenen Herstellungsverfahren hergestellt.

Verbindung 4 wurde wie folgt erhalten:

In ein Gemisch aus 12 g (174 mMol) 1,2,4-Triazol und 1,4 g (12,5 mMol) Kalium-tert.-butylat in 30 ml absolutem Dimethylformamid wird unter Stickstoffatmosphäre und unter Rühren eine Lösung von 15,9 g (54 mMol) 2-(2,4-Difluorphenyl)-2-[1-(trifluormethylmercapto)-cycloprop-1-yl]-oxiran in 20 ml absolutem Dimethylformamid eingetropft. Das Reaktionsgemisch wird 8 Stunden bei 100° C gerührt. Danach zieht man das Lösungsmittel unter vermindertem Druck ab, nimmt den Rückstand in Essigsäureethylester auf, wäscht zweimal mit Wasser und engt nach dem Trocknen über Natriumsulfat unter vermindertem Druck ein. Der Rückstand wird mit Chloroform als Laufmittel über eine Kieselgelsäule chromatographiert. Man erhält auf diese Weise 12,5 g (65 % der Theorie) an 1-(2,4-Difluorphenyl)-1-hydroxy-2-(1,2,4-triazol-1-yl)-1-(1-trifluormethylmercapto-cycloprop-1-yl)-ethan vom Schmelzpunkt 103° C.

Herstellung der für die Verbindung 4 verwendeten Ausgangsverbindungen:

a) 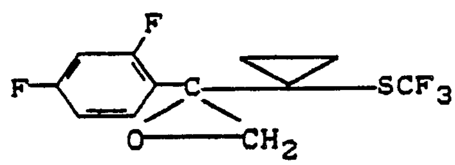

In ein Gemisch von 1,9 g (63 mMol) Natriumhydrid (80 %ig) und 13,5 g (61 mMol) Trimethyloxosulfoniumiodid werden bei 10° C unter Stickstoffatmosphäre 50 ml absolutes Dimethylsulfoxid getropft. Das Gemisch wird noch eine Stunde bei 10° C gerührt und dann bei der gleichen Temperatur tropfenweise mit einer Lösung von 15,3 g (54 mMol) 2,4-Difluorphenyl-(1-trifluormethylmercapto-cycloprop-1-yl)-keton in 20 ml abolutem Dimethylsulfoxid versetzt. Man rührt 48 Stunden bei Raumtemperatur und anschließend 1 Stunde bei 40° C. Das Reaktionsgemisch wird auf Wasser gegossen. Man extrahiert mit Essigsäureethylester, wäscht die vereinigten organischen Phasen mit Wasser, trocknet über Natriumsulfat und engt unter vermindertem Druck ein. Man erhält 15,9 g (99 % der Theorie) an 2-(2,4-Difluorphenyl)-2-[1-(trifluormethylmercapto)-cycloprop-1-yl]-oxiran in Form eines farblosen Öles.

$^1$H-NMR (360 MHz; $CDCl_3$):

$\delta$ = 1,00-1,50 (m, 4H); 3,04 (m,2H);
6,80-6,96 (m, 2H); 7,38-7,48 (m,1H).

b) 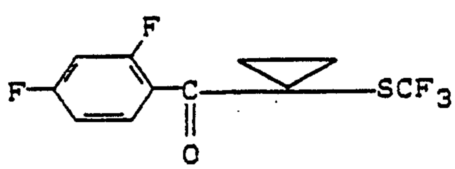

Eine Lösung von 47 g (0,15 Mol) (3-Chlor-1-trifluormethylmercapto-propyl)-(2,4-difluorphenyl)-keton in 70 ml tert.-Butanol wird bei 40°C unter Rühren in ein Gemisch aus 20 g (0,18 Mol) Kalium-tert.-butylat und 50 ml tert.-Butanol getropft. Das Reaktionsgemisch wird 4 Stunden bei 40°C gerührt und dann auf Wasser gegossen. Man extrahiert mit Essigsäureethylester, wäscht die vereinigten organischen Phasen mit Wasser, trocknet über Natriumsulfat und engt durch Abziehen des Lösungsmittels unter vermindertem Druck ein. Der verbleibende Rückstand wird über eine Kieselgelsäule mit Petrolether: Methylenchlorid = 1:0,4 als Laufmittel chromatographiert. Man erhält 15,3 g (36 % der Theorie) 2,4-Difluorphenyl-(1-trifluormethylmercapto-cycloprop-1-yl)-keton in Form eines farblosen Öles.

$^1$H-NMR (200 MHz; $CDCl_3$):

$\delta$ = 1,57 (m, 2H); 1,95 (m, 2H);

6,80-7,02 (m, 2H); 7,55-7,68 (m, 1H).

c) 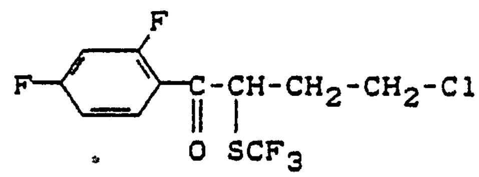

In eine Lösung von 43 g (0,2 Mol) 3-Chlorpropyl-2,4-difluorphenyl-keton in 100 ml Methylenchlorid werden bei 20 bis 25°C unter Rühren 75 g (0,55 Mol) Trifluormethylsulfenylchlorid gegeben. Das Gemisch wird 108 Stunden bei Raumtemperatur gerührt und dann durch Abziehen des Lösungsmittels unter vermindertem Druck eingeengt. Man erhält 63 g (3-Chlor-1-trifluormethylmercapto-propyl)-2,4-difluorphenyl-keton.

$^{19}$F-NMR (Externer Standard: $CF_3COOH$):

$\delta$ = - 36,6 ppm (Duplett)

\+ 24,5 ppm (Multiplett)

\+ 27,9 ppm (Multiplett)

d) 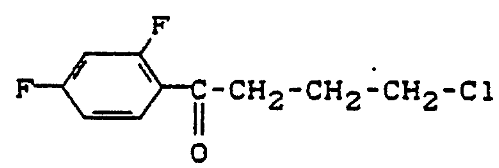

In ein Gemisch von 50 g (0,44 Mol) 1,3-Difluorbenzol und 64 g (0,48 Mol) wasserfreiem Aluminiumchlorid werden bei 20°C unter Rühren 62 g (0,44 Mol) 4-Chlorbuttersäurechlorid getropft. Das Reaktionsgemisch wird 3,5-Stunden bei 30°C gerührt bis eine klare Lösung entstanden ist und die Gasentwicklung nachgelassen hat. Nach dem Abkühlen auf Raumtemperatur werden 300 ml Methylenchlorid zugegeben. Man gießt die entstandene Lösung auf 300 g Eis, trennt die organische Phase ab, trocknet über Natriumsulfat und engt durch Abziehen des Lösungsmittels unter vermindertem Druck ein. Man erhält 83 g (86 % der Theorie) an 3-Chlorpropyl-2,4-difluorphenyl-keton in Form einer Flüssigkeit.

Kp = 80°C / 0,1 mbar

Verbindung 9 wurde wie folgt erhalten:

Unter Stickstoffatmosphäre werden 65 g (0,94 Mol) 1,2,4-Triazol und 71 g (0,63 Mol) Kalium-tert.-butylat in 160 ml absolutem Dimethylformamid vorgelegt und auf 80°C erwärmt. Bei dieser Temperatur tropft man unter Rühren eine Lösung von 70,6 g (0,26 Mol) 1-Chlor-2-(1-chlorcyclopropyl)-3-(4-fluorphenyl)-propan-2-ol in 90 ml absolutem Dimethylformamid hinzu. Es wird 6 Stunden bei 100°C nachgerührt und dann durch Abziehen des Verdünnungsmittels unter vermindertem Druck eingeengt. Man nimmt den Rückstand in Essigester auf, wäscht mit Wasser und zieht nach dem Trocknen über Natriumsulfat das Lösungsmittel unter vermindertem Druck ab. Das verbleibende Produkt wird über eine Kieselgel-Säule mit Chloroform als Laufmittel chromatographiert. Man erhält auf diese Weise 28,2 g (39 % der Theorie) an 1-(4-Fluorphenyl)-2-(1-chlor-cyclopropyl)-3-(1,2,4-triazol-1-yl)-propan-2-ol in Form einer Festsubstanz vom Schmelzpunkt 111°C.

Analog wurden die Verbindungen 7 und 8 hergestellt.

Herstellung der für die Herstellung der Verbindung 9 verwendeten Ausgangsverbindungen:

a) 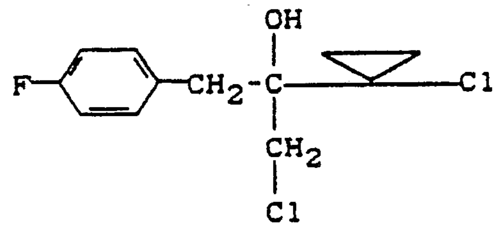

Eine Lösung von 65,7 g (0,35 Mol) 4-Fluorbenzyl-bromid in 430 ml absolutem Diethylether wird bei Raumtemperatur in ein Gemisch aus 9,3 g (0,38 Mol) Magnesium-Spänen in 185 ml Diethylether getropft. Man erhitzt 30 Minuten unter Rückfluß und tropft die entstandene Lösung dann bei 30 °C unter Rühren in eine Lösung aus 47,8 g (0,32 Mol) 1-Chlor-1-chloracetyl-cyclopropan in 300 ml Ether ein. Das Reaktionsgemisch wird 4 Stunden bei -30 °C gerührt. Danach tropft man eine Lösung von 31 ml Essigsäure in 300 ml Diethylether zu. Das entstandene Reaktionsgemisch wird auf 1.200 ml Wasser gegossen. Die organische Phase wird abgetrennt, mit wäßriger Natriumhydrogensulfit-Lösung und mit Wasser gewaschen, über Natriumsulfat getrocknet und unter vermindertem Druck eingeengt. Man erhält 70,6 g (86 % der Theorie) an 1-Chlor-2-(1-chlor-cyclopropyl)-3-(4-fluor-phenyl)-propan-2-ol in Form eines öligen Produktes.
$^1$H-NMR (200 MHz, $CDCl_3$): $\delta$ = 0,6-1,2 (m, 4H); 3,05 (d, 1H); 3,15 (d 1H); 3,75 (d, 1H); 4,03 (d, 1H); 7,0 (t, 2H); 7,22-7,37 (m, 2H).

b) 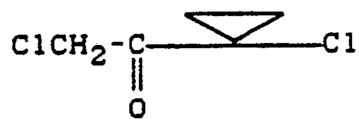

Bei Raumtemperatur werden 40,5 ml (0,5 Mol) Sulfurylchlorid unter Rühren langsam in eine Lösung von 54 g (0,46 Mol) 1-Acetyl-1-chlor-cyclopropan in 250 ml Methylenchlorid eingetropft. Es wird zunächst 14 Stunden bei Raumtemperatur und dann 30 Minuten bei 30 °C gerührt. Das Reaktionsgemisch wird dann nacheinander mit gesättigter, wäßriger Natriumhydrogencarbonat-Lösung und mit Wasser gewaschen. Danach wird die organische Phase über Natriumsulfat getrocknet und unter vermindertem Druck eingeengt. Man erhält auf diese Weise 51,5 g (74 % der Theorie) an 1-Chlor-1-chloracetyl-cyclopropan in Form einer öligen Substanz.
$^1$H-NMR (60 MHz, $CDCl_3$): $\delta$ = 1,2-1,9 (m, 4H); 4,8 (s, 2H).

Verbindung 10 wurde wie folgt erhalten:

In ein Gemisch aus 10 g Kaliumcarbonat, 15 g (0,22 Mol) 1,2,4-Triazol und 50 ml Acetonitril wird unter Stickstoffatmosphäre eine Lösung von 13,4 g (0,05 Mol) 2-(1-Chlorcyclopropyl)-2-(4-chlorphenylethyl)-oxiran in 20 ml Acetonitril eingetropft, wobei das Reaktionsgemisch unter Rückfluß erhitzt wird. Nach beendeter Zugabe rührt man das Reaktionsgemisch noch 8 Stunden unter Rückfluß, saugt dann vom Rückstand ab und engt das Filtrat durch Abziehen des Lösungsmittels unter vermindertem Druck ein. Man nimmt den verbleibenden Rückstand in Essigester auf, wäscht die organische Phase mit Wasser, trocknet über Natriumsulfat und engt durch Abziehen des Lösungsmittels unter vermindertem Druck ein. Der anfallende Rückstand wird mit Chloroform/Ethanol = 98:2 als Laufmittel über eine Kieselgel-Säule chromatographiert. Nach Umkristallisation des dabei verbleibenden Produktes aus Cyclohexan erhält man 5,1 g (30 % der Theorie) an 2-(1-Chlorcyclopropyl)-4-(4-chlorphenyl)-1-(1,2,4-triazol-1-yl)-butan-2-ol in Form einer Festsubstanz vom Schmelzpunkt 137 °C.

Analog wurden die Verbindungen 11, 12, 14 und 18 hergestellt.

Die für die Herstellung der Verbindung 10 verwendeten Ausgangsverbindungen wurden wie folgt erhalten:

a)

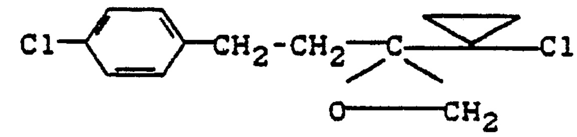

16 ml (0,22 Mol) Dimethylsulfid und 24,2 g (0,19 Mol) Dimethylsulfat werden in 30 ml tert.-Butanol gegeben und 14 Stunden bei 20° C stehengelassen. Unter Rühren tropft man in das Reaktionsgemisch zunächst eine Lösung von 17 g (0,07 Mol) 1-Chlorcyclopropyl-4-chlorphenylethylketon in 70 ml tert.-Butanol und trägt dann 22 g Kaliumhydroxid-Pulver ein, wobei die Temperatur des Reaktionsgemisches auf 20 bis 30° C gehalten wird. Man rührt noch 3 Stunden bei 30° C, zieht dann das Dimethylsulfid unter vermindertem Druck ab und gießt das Reaktionsgemisch danach auf 50 ml einer 1 %igen wäßrigen Wasserstoffperoxid-Lösung. Das Gemisch wird mit Essigester extrahiert. Die organische Phase wird mit Wasser gewaschen und nach dem Trocknen über Natriumsulfat durch Abziehen des Lösungsmittels unter vermindertem Druck eingeengt. Man erhält auf diese Weise 13,4 g (75 % der Threorie) an 2-(1-Chlorcyclopropyl)-2-(4-chlorphenylethyl)-oxiran in Form eines öligen Produktes, das ohne zusätzliche Reinigung weiter umgesetzt wird.

b)

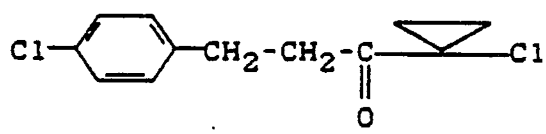

In einen 100 ml Autoklaven werden 460 mg (0,5 mmol) Tris-triphenylphosphin-rhodiumchlorid (= 1 Mol-%, bezogen auf die Reaktionskomponenten) gegeben. Nach dem Spülen mit Stickstoff wird eine luftfreie Lösung von 12 g (0,05 Mol) 1-Chlorcyclopropyl-4-chlorphenyl-ethenyl-keton in 40 ml Toluol hinzugefügt und unter einem Wasserstoffdruck von 30 bar auf 50° C erhitzt. Der Wasserstoffdruck wird zwischen 40 und 50 bar gehalten bis die Gasaufnahme beendet ist (nach etwa 1 Stunde). Anschließend läßt man 1 Stunde nachreagieren. Zur Aufarbeitung wird das Lösungsmittel unter vermindertem Druck abgezogen und der verbleibende Rückstand mit Dichlormethan als Laufmittel an Kieselgel gereinigt. Man erhält 11 g (90 % der Theorie) an 1-Chlorcyclopropyl-4-chlorphenylethyl-keton in Form eines öligen Produktes.

$^1$H-NMR (200 MHz, $CDCl_3$):

δ = 1,28-1,38 (m, 2H), 1,57-1,67 (m, 2H), 2,84 (t, 2H), 3,15 (t, 2H), 7,08-7,29 (m, 4H).

c)

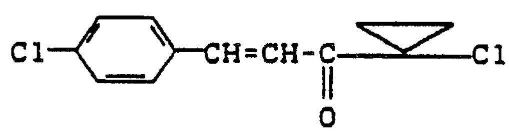

In ein Gemisch von 60 g (0,5 Mol) 1-Chlorcyclopropylmethyl-keton, 70 g (0,5 Mol) 4-Chlorbenzaldehyd und 250 ml Ethanol werden bei Raumtemperatur 50 ml Wasser und 10 Plätzchen festes Natriumhydroxid gegeben. Es wird 16 Stunden bei Raumtemperatur gerührt. Danach wird der ausgefallene Feststoff angesaugt. Man erhält auf diese Weise 108,5 g (89 % der Theorie) an 1-Chlorcyclopropyl-4-chlorphenyl-ethenyl-keton in Form einer Festsubstanz vom Schmelzpunkt 92° C.

Verbindung 13 wurde wie folgt erhalten:

In ein Gemisch aus 29 g (0,42 Mol) 1,2,4-Triazol, 19 g (0,14 Mol) Kaliumcarbonat und 100 ml Acetonitril wird unter Stickstoffatmosphäre eine Lösung 27,9 g (0,11 Mol) 2-(1-Chlorcyclopropyl)-2-(4-chlor-phenoxymethyl)-oxiran in 50 ml Acetonitril eingetropft, wobei das Reaktionsgemisch unter Rückfluß gekocht wird. Nach beendeter Zugabe wird noch weitere 8 Stunden unter Rückfluß erhitzt. Anschließend saugt man vom festen Rückstand ab und engt das Filtrat unter vermindertem Druck ein. Der verbleibende Rückstand wird in Essigester aufgenommen. Die dabei entstehende Lösung wird mit Wasser gewaschen, über Natriumsulfat getrocknet und unter vermindertem Druck eingeengt. Das so erhaltene Produkt wird auf chromatographischem Wege über Kieselgel mit Chloroform/Ethanol = 97:3 als Laufmittel gereinigt. Man erhält auf diese Weise 13,7 g (39% der Theorie) an 2-(1-Chlorcyclopropyl)-1-(4-chlorphenoxy)-3-(1,2,4-triazol-1-yl)-propan-2-ol in Form eine Öles.

$^1$H-NMR (80 MHz, $CDCl_3$):

δ = 0,5-1,4(m,4H), 4,1-4,4(m,3H),

4,75 (s,2H), 6,8-7,25 (m, 4H),
8,0 (s,1H), 8,17 (s, 1H).

Die für die Herstellung der Verbindung 13 verwendeten Ausgangsverbindungen wurden wie folgt erhalten:

a)

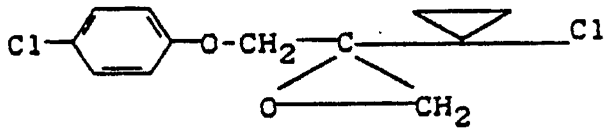

In ein Gemisch aus 3,9 g (0,13 Mol) Natriumhydrid (80%ig) und 27 g (0,12 Mol) Trimethyloxosulfonium-iodid werden bei 10 °C unter Stickstoffatmosphäre und unter Rühren 90 ml absolutes Dimethylsulfoxid zugetropft. Man rührt noch 1 Stunde bei Raumtemperatur, kühlt dann auf 10 °C ab und tropft eine Lösung von 27 g (0,11 Mol) 1-Chlorcyclopropyl-4-chlorphenoxymethyl-keton in 40 ml absolutem Dimethylsulfoxid hinzu. Das Reaktionsgemisch wird zunächst 48 Stunden bei 20 °C gerührt, dann 1 Stunde auf 40 °C erwärmt und danach auf Wasser gegossen. Das entstehende Gemisch wird mit Essigester extrahiert. Die organische Phase wird mit Wasser gewaschen, über Natriumsulfat getrocknet und unter vermindertem Druck eingeengt. Man erhält auf diese Weise 27,9 g (98% der Theorie) an 2-(1-Chlorcyclopropyl)-2-(4-chlorpenoxymethyl)-oxiran in Form eines Öles.

$^{1}$H-NMR (60 $MH_z$, $CDCl_3$):
$\delta$ = 0,75 - 1,4 (m, 4H), 2,8 (d, 1H), 3,05 (d, 1H), 4,25 (d, 1H), 4,58 (d, 1H), 6,8 - 7,4 (m, 4H).

b)

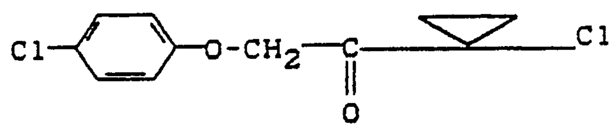

In eine Lösung von 51 g (0,33 Mol) 1-Chlor-1-chloracetyl-cyclopropan in 250 ml Acetonitril werden nacheinander 49 g (0,38 Mol) 4-Chlorphenol und 70 g (0,51 Mol) Kaliumcarbonat gegeben. Das Gemisch wird 8 Stunden unter Rückfluß erhitzt, dann filtriert und eingeengt durch Abziehen des Lösungsmittels. Man nimmt den Rückstand in Essigester auf, wäscht nacheinander mit verdünnter, wäßriger Natronlauge und Wasser, trocknet über Natriumsulfat und zieht unter vermindertem Druck das Lösungsmittel ab. Der Rückstand wird einer Destillation unterworfen. Man erhält auf diese Weise 27 g (33% der Theorie) an 1-Chlor-cyclopropyl-4-chlorphenoxy-methyl-keton in Form einer Flüssigkeit vom Siedepunkt 125 -127 °C / 0,1 mbar.

Verbindung 19 wurde wie folgt erhalten:

In ein Gemisch aus 2,5 g Kaliumtertiärbutylat, 26 g (0,38 Mol) 1,2,4-Triazol und 150 ml Dimethylformamid wird unter Stickstoffatmosphäre eine Lösung von 36,5 g (0,13 Mol) 2-(1-Chlorcyclopropyl)-2-(2,4-dichlorphenylethylen)-oxiran in 20 ml Dimethylformamid eingetropft.

Nach beendeter Zugabe rührt man das Reaktionsgemisch noch 8 Stunden unter Rückfluß und engt das Filtrat durch Abziehen des Lösungsmittels unter vermindertem Druck ein. Man nimmt den verbleibenden Rückstand in Essigester auf, wäscht die organische Phase mit Wasser, trocknet über Natriumsulfat und engt durch Abziehen des Lösungsmittels unter vermindertem Druck ein. Der anfallende Rückstand wird mit Methylenchlorid/Essigsäureethylester (4:1) als Laufmittel über eine Kieselgel-Säure chromatographiert.

Man erhält 13,8 g (30 % der Theorie) an 2-(1-Chlorcyclopropyl)-4-(3,4-dichlorphenyl)-1-(1,2,4-triazol-1-yl)-but-3-en-2-ol in Form einer Festsubstanz vom Schmelzpunkt 140 °C.

Analog wurden die Verbindungen 15, 16 und 17 hergestellt.

Die für die Herstellung der Verbindung 19 verwendeten Ausgangsverbindungen wurden wie folgt erhalten:

a)

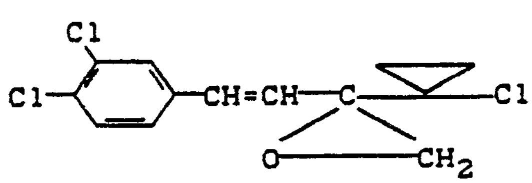

36,5 g (0,59 Mol) Dimethylsulfid und 88 g (0,70 Mol) Dimethylsulfat werden in 100 ml tert.-Butanol gegeben und über Nacht bei 20 °C stehengelassen. Unter Rühren tropft man in das Reaktionsgemisch zunächst eine Lösung von 54 g (0,19 Mol) 1-Chlorcyclopropyl-3,4-dichlorphenylethenyl-keton in 70 ml tert.-Butanol und trägt dann 59 g Kaliumhydroxid-Pulver ein, wobei die Temperatur des Reaktionsgemisches auf 20 bis 30 °C gehalten wird. Man rührt noch 6 Stunden bei 30 °C, zieht dann das Dimethylsulfid unter vermindertem Druck ab und gießt das Reaktionsgemisch danach auf 50 ml einer 1 %igen wäßrigen Wasserstoffperoxid-Lösung. Das Gemisch wird mit Essigester extrahiert. Die organische Phase wird mit Wasser gewaschen und nach dem Trocknen über Natriumsulfat durch Abziehen des Lösungsmittels unter vermindertem Druck eingeengt. Man erhält auf diese Weise 37,2 g (65 % der Theorie) an 2-(1-Chlorcyclopropyl)-2-(3,4-dichlorphenylethenyl)-oxiran in Form eines öligen Produktes, das ohne zusätzliche Reinigung weiter umgesetzt wird.

b)

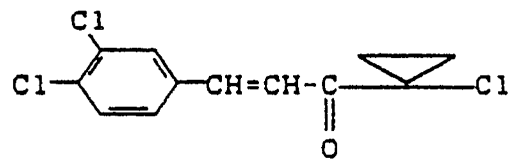

In ein Gemisch von 65 g (0,55 Mol) 1-Chlorcyclopropylmethyl-keton, 84 g (0,48 Mol) 3,4-Dichlorbenzaldehyd und 290 ml Ethanol werden bei Raumtemperatur 60 ml Wasser und 22 Plätzchen festes Natriumhydroxid gegeben. Es wird 16 Stunden bei Raumtemperatur gerührt. Danach wird der ausgefallene Feststoff abgesaugt. Man erhält auf diese Weise 114 g (85 % der Theorie) an 1-Chlorcyclopropyl-3,4-dichlorphenylethenyl-keton in Form einer Festsubstanz vom Schmelzpunkt 96 °C.

Beispiel 1

Zum Nachweis der Wirksamkeit gegen Pilze werden die minimalen Hemm-Konzentrationen (MHK) von erfindungsgemäßen Wirkstoffen bestimmt:

Ein Agar, der aus Bierwürze und Pepton hergestellt wird, wird mit erfindungsgemäßen Wirkstoffen in Konzentrationen von 0,1 mg/1 bis 5000 mg/l versetzt. Nach Erstarren des Agars erfolgt Kontamination mit Reinkulturen der in der Tabelle aufgeführten Testorganismen. Nach 2-wöchiger Lagerung bei 28 °C und 60 bis 70 % rel. Luftfeuchtigkeit wird die MHK bestimmt. MHK ist die niedrigste Konzentration an Wirkstoff, bei der keinerlei Bewuchs durch die verwendete Mikrobenart erfolgt; sie ist in der nachstehenden Tabelle 1 angegeben.

<u>Tabelle 1</u> MHK's in mg/l bei der Einwirkung der erfindungsgemäß zu verwendenden Verbindungen auf Pilze

| Testorganismen | Verbindungen | | | | | | | | | | | | |
|---|---|---|---|---|---|---|---|---|---|---|---|---|---|
| | 1 | 2 | 3*) | 4 | 5*) | 6 | 7 | 8 | 9 | 10 | 11 | 12 | 13 |
| Alternaria tenuis | 50 | | 350 | 75 | 20 | 75 | | | 150 | 750 | 750 | | 500 |
| Aspergillus niger | 50 | 75 | 75 | 35 | 15 | 100 | <20 | <20 | 15 | 10 | 50 | 100 | 35 |
| Aureobasidium pullulans | 10 | | 15 | 20 | 5 | 15 | | | 15 | 50 | 100 | | 150 |
| Chaetomium globosum | 20 | <20 | 35 | 5 | 35 | 15 | <20 | <20 | 2 | 20 | 50 | 50 | 50 |
| Cladosporium cladosporioides | 20 | | 75 | 100 | 15 | 35 | | | 35 | 50 | 500 | | 500 |
| Lentinus tigrinus | 15 | | 35 | 100 | 75 | 20 | | | 15 | 10 | 20 | | 20 |
| Penicillium glaucum | 200 | 150 | 350 | 75 | 75 | 200 | <20 | 200 | 350 | 35 | 100 | 50 | 150 |
| Sclerophoma pityophila | 2 | | 5 | 2 | 0,75 | 2 | | | 1,5 | 3,5 | 15 | | 15 |

*) in Form der Saccharin-Additionsverbindung

Tabelle 1 (Fortsetzung)

| Testorganismen | Verbindungen | | | | | |
|---|---|---|---|---|---|---|
| | 14 | 15 | 16 | 17 | 18 | 19 |
| Alternaria tenuis | 500 | 50 | 50 | 100 | 500 | 150 |
| Aspergillus niger | 50 | 20 | 20 | 20 | 10 | 10 |
| Aureobasidium pullulans | 50 | 20 | 10 | 10 | 50 | 100 |
| Chaetomium globosum | 50 | 10 | 50 | 50 | 10 | 10 |
| Cladosporium cladosporioides | 50 | 5 | 5 | 20 | 20 | 20 |
| Lentinus tigrinus | 20 | 20 | 7,5 | 15 | 10 | 5 |
| Penicillium glaucum | 100 | 200 | 20 | 150 | 35 | 100 |
| Sclerophoma pityophila | 2 | 0,5 | 2 | 2 | 2 | 2 |

Beispiel 2

Eine Mischkultur von Grün-, Blau-, Braun- und Kieselalgen (Stichococcus bacillaris Naegeli, Euglena gracilis Klebs, Chlorella pyrenoidosa Chick, Phormidium foveolarum Gomont, Oscillatoria geminata Meneghini und Phae odactylum tricornutum Bohlin) wird unter Durchperlen von Luft in Allens Nährlösung (Arch. Mikrobiol. 17, 34 bis 53 (1952)), die auf 4 l steriles Wasser 0,2 g Ammoniumchlorid, 4,0 g Natriumnitrat, 1,0 g Dikaliumhydrogenphosphat, 0,2 g Calciumchlorid, 2,05 g Magnesiumsulfat und 0,02 g Eisenchlorid enthält, gegeben. Nach 2 Wochen ist die Nährlösung durch intensives Algenwachstum tief grün-blau gefärbt. Das Absterben der Algen nach Zugabe erfindungsgemäßer Wirkstoffe erkennt man an dem Entfärben der Nährlösung.

Tabelle 2

| Algen-abtötende Konzentrationen (mg/l) der unten angegebenen Substanzen | |
|---|---|
| Verbindung | abtötende Konzentration in mg/l |
| 9 | ≦ 100 |

Beispiel 3

Bestimmung der toxischen Grenzwerte (kg/m³ Holz) der erfindungsgemäßen Wirkstoffe für Coniophora puteana bzw. Polyporus versicolor auf Kiefern- bzw. Buchenholz

Die Bestimmung der toxischen Grenzwerte erfolgt in Anlehnung an die von H.P. Sutter, Int. Biodeterioration Bulletin 14 (3), 1978, Seiten 95 bis 99, beschriebene Methode.

Für die Tests werden jeweils frisch geschnittene, dünne Stirnholzstücke (Größe 40 x 40 mm, Dicke etwa 2 mm) im Vakuum mit Lösungen unterschiedlicher Wirkstoffkonzentrationen getränkt, und zwar jeweils 15 Holzproben mit der Lösung einer bestimmten Wirkstoffkonzentration. Von diesen 15 Proben werden jeweils 5 für einen mykologischen Test verwendet.

Die Menge des absorbierten Wirkstoffes wird aus der Lösungsmittelretention (die durch Wiegen des Holzstückchens vor und nach der Imprägnierung bestimmt wird), der Holzdichte und der Konzentration des Wirkstoffes in der zurückbleibenden Imprägnierlösung bestimmt.

Vor dem mykologischen Test werden die Prüflinge mit Propylenoxid sterilisiert und je 1 Prüfling in einer Petrischale in Kontakt mit dem voll entwickelten Mycel des Testpilzes auf Malzextraktagar gebracht. Nach 6 Wochen bei 21 bis 23 °C werden visuell die Toxizitätsgrenzen festgestellt.

In der folgenden Tabelle sind die Toxizitätsgrenzen (kg/m³ Holz) für erfindungsgemäße Substanzen angegeben; die Toxizitätsgrenzen geben die Konzentrationen an, bei der das Holz gerade noch angegriffen (unterer Grenzwert) und bei der das Holz nicht mehr angegriffen wird (oberer Grenzwert).

Tabelle 3

| Toxizitätsgrenzen (kg/m³ Holz) erfindungsgemäßer Wirkstoffe für holzzerstörende Pilze | | |
|---|---|---|
| Verbindung | Coniophora puteana auf Kiefernholz | Polyporus versicolor auf Buchenholz |
| 3 | 0,12 - 0,23 | < 0,04 |
| 5 | 0,25 - 0,76 | 0,10 - 0,16 |
| 6 | 0,44 - 1,13 | 0,09 - 0,20 |
| 9 | 0,26 - 0,76 | < 0,23 |

Beispiel 4 (Wirkung erfindungsgemäß fungizid ausgerüsteter Kunststoffe gegen Mikroorganismen)

Die Bestimmung der Wirkung, die die erfindungsgemäß fungizid ausgerüsteten Kunststoffe gegen Mikroorganismen (verschiedene Pilzgattungen) aufweisen, wurde gemäß der Schweizer Prüfnorm SNV No. 195 921 (von 1976; "Prüfung der antimykotischen Wirkung von ausgerüsteten Textilien und anderen Materialien mit Hilfe des Agardiffusionstestes") vorgenommen.

Für die Bestimmung wurden Prüfkörper (Scheiben; Durchmesser: 3 cm; Dicke je nach zu prüfendem Kunststoff: 250 µm bis 5 mm) der fungizid ausgerüsteten Kunststoffe und -zur Kontrolle - der entsprechenden nicht ausgerüsteten Kunststoffe auf 2-Schicht Nähragarplatten gelegt, die mit Sporen oder Hyphen des jeweiligen Testorganismus beimpft worden waren. Die Platten wurden 4 Wochen bei 29 ± 1 °C bebrütet. Anschließend wurde der Pilzbewuchs auf den Prüfkörpern und in der Kontaktzone unter den Prüfkörpern visuell bestimmt und, sofern sich eine Hemmzone unter den Prüfkörpern ausgebildet hatte, die Größe dieser Hemmzone durch Messung bestimmt.

Als Hemmzone wird die pilzfreie Zone bezeichnet, die nach folgender Formel berechnet wird:

$$H = \frac{D - d}{2},$$

in der

H = Hemmzone [mm]

D = Gesamtdurchmesser von Prüfkörper und Hemmzone [mm];

d = Durchmesser des Prüfkörpers [mm]

bedeuten.

In der nachstehenden Tabelle 4 sind die bei der Auswertung der Versuche erhaltenen Ergebnisse, Pilzbewuchs und Größe der Hemmzonen, angegeben, die bei Verwendung der in der Tabelle angegebenen Verbindungen in den ebenfalls in der Tabelle angegebenen Mengen in den ebenfalls in der Tabelle angegebenen Kunststoffen erhalten wurden.

Tabelle 4

Wirkung der fungizid ausgerüsteten Kunststoffe gegen verschiedene Testorganismen

Verwendete Testorganismen:

(a) Aspergillus niger
(b) Aspergillus terreus
(c) Chaetomium globosum
(d) Trichoderma viride
(e) Cladosporium herbarium
H = Hemmzone [mm]
PB = Pilzbewuchs (4 Benotungen: kein, schwach, mittel, voll)

| als Fungizid verwendete Verbindung | angewendete Menge [g/100 g Kunststoff] | (a) H/PB | (b) H/PB | (c) H/PB | (d) H/PB | (e) H/PB |
|---|---|---|---|---|---|---|
| 7 | 0,1/Polyvinylchlorid | 15-18/kein | 15-18/kein | 12-15/kein | 0/kein | 15-18/kein |
| 3 | 0,4/Polyvinylchlorid | 4-8/kein | 1-3/kein | 2-3/kein | 0/schwach | 10-13/kein |
| 8 | 0,6/Polyvinylchlorid | 0/schwach | 13-15/kein | 18-25/kein | 0/kein | 5-8/kein |
| Kontrolle | -/Polyvinylchlorid | 0/voll | 0/voll | 0/voll | 0/voll | 0/voll |
| 7 | 0,1/Polymethan | 15-17/kein | 16-20/kein | 17-20/kein | 0/kein | 15-18/ |
| Kontrolle | -/Polymethan | 0/voll | 0/voll | 0/voll | 0/voll | 0/voll |

## Ansprüche

1. Verwendung von Derivaten des Triazolylmethylcyclopropyl-carbinols der Formel

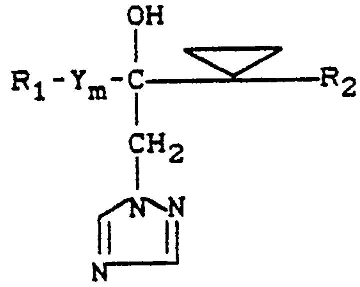

(I)

in der
$R_1$ eine gegebenenfalls substituierte Alkyl-, Cycloalkyl- oder Arylgruppe und
Y eine $-CH_2-$, $-OCH_2-$, $-CH_2-CH_2-$ oder -CH=CH-Gruppe bedeuten,
m für 0 oder 1 steht und
$R_2$ Halogen oder die Gruppe $ZR_4$ bedeutet, in der Z für Sauerstoff, Schwefel, eine Sulfinyl-oder Sulfonylgruppe steht und $R_4$ ein gegebenenfalls durch Halogen substituierter Alkyl-, Cycloalkyl-, Aralkyl-oder Aryl-Rest ist,
als Mikrobizide zum Schutz technischer Materialien.

2. Verwendung gemäß Anspruch 1, dadurch gekennzeichnet, daß in Formel (I)
$R_1$ ein gegebenenfalls durch Halogen, Alkyl oder Halogenalkyl substituierter Phenyl-, Biphenyl-oder Naphthylrest ist,
$R_2$ für Halogen oder eine gegebenenfalls durch Halogen substituierte Alkylmercapto-Gruppe steht und
Y und m die unter Formel (I) angegebene Bedeutung haben.

3. Verwendung gemäß Ansprüchen 1 oder 2 als Fungizide zum Schutz von Holz vor Holz dauerhaft verfärbenden oder holzzerstörenden Pilzen und in Anstrichmitteln.

4. Mikrobizide Mittel für den Materialschutz enthaltend als Wirkstoff Derivate des Triazolylmethylcyclopropyl-carbinols der Formel

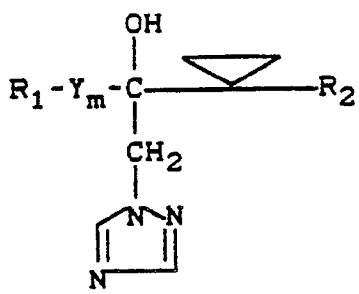

(I),

in der
$R_1$ eine gegebenenfalls substituierte Alkyl-, Cycloalkyl- oder Arylgruppe und
Y eine $-CH_2-$, $-OCH_2-$, $-CH_2-CH_2-$ oder -CH=CH-Gruppe bedeuten,
m für 0 oder 1 steht und
$R_2$ Halogen oder die Gruppe $ZR_4$ bedeutet, in der Z für Sauerstoff, Schwefel, eine Sulfinyl-oder Sulfonylgruppe steht und $R_4$ ein gegebenenfalls durch Halogen substituierter Alkyl-, Cycloalkyl-, Aralkyl-oder Aryl-Rest ist,
übliche Formulierungshilfsmittel und gegebenenfalls bekannterweise im Materialschutz verwendete Mikrobizide.